(19)

**Europäisches Patentamt**
**European Patent Office**
**Office européen des brevets**

(11) **EP 2 921 990 B1**

(12) **EUROPEAN PATENT SPECIFICATION**

(45) Date of publication and mention
of the grant of the patent:
**06.07.2016 Bulletin 2016/27**

(51) Int Cl.:
*G06K 9/00* *(2006.01)*    *G06K 9/62* *(2006.01)*

(21) Application number: **15000801.9**

(22) Date of filing: **18.03.2015**

(54) **METHOD AND APPARATUS FOR UNSUPERVISED SEGMENTATION OF MICROSCOPIC COLOR IMAGE OF UNSTAINED SPECIMEN AND DIGITAL STAINING OF SEGMENTED HISTOLOGICAL STRUCTURES**

VERFAHREN UND VORRICHTUNG FÜR EINE UNÜBERWACHTE SEGMENTIERUNG VON MIKROSKOPISCHEN FARBBILDERN VON UNGEFLECKTER PROBE UND DIGITALES ANFÄRBEN VON SEGMENTIERTEN HISTOLOGISCHEN STRUKTUREN

PROCÉDÉ ET APPAREIL DE SEGMENTATION NON SUPERVISÉE D'UNE IMAGE COULEUR MICROSCOPIQUE D'UN SPÉCIMEN NON COLORÉ ET COLORATION NUMÉRIQUE DE STRUCTURES HISTOLOGIQUES SEGMENTÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.03.2014 US 201414221017**

(43) Date of publication of application:
**23.09.2015 Bulletin 2015/39**

(73) Proprietor: **RUDJER BOSKOVIC INSTITUTE**
**10000 Zagreb (HR)**

(72) Inventors:
 • **Kopriva, Ivica**
  **10000 Zagreb (HR)**
 • **Popovic-Hadzija, Marijana**
  **10000 Zagreb (HR)**
 • **Hadzija, Mirko**
  **10000 Zagreb (HR)**
 • **Aralica, Gorana**
  **10000 Zagreb (HR)**

(74) Representative: **Vukmir, Mladen et al**
 **Vukmir & Associates**
 **Gramaca 2L**
 **10000 Zagreb (HR)**

(56) References cited:
**US-A1- 2007 053 573**

 • **ANNE-SOPHIE MONTCUQUET ET AL: "In Vivo
Fluorescence Spectra Unmixing and
Autofluorescence Removal by Sparse
Nonnegative Matrix Factorization", IEEE
TRANSACTIONS ON BIOMEDICAL
ENGINEERING, vol. 58, no. 9, 1 September 2011
(2011-09-01), pages 2554-2565, XP055013159,
ISSN: 0018-9294, DOI:
10.1109/TBME.2011.2159382**
 • **MUNOZ-BARRUTIA A ET AL: "Blind Spectral
Unmixing of M-FISH Images by Non-negative
Matrix Factorization", 2007 ANNUAL
INTERNATIONAL CONFERENCE OF THE IEEE
ENGINEERING IN MEDICINE AND BIOLOGY
SOCIETY : [EMBC '07] ; LYON, FRANCE, 22 - 26
AUGUST 2007 ; [IN CONJUNCTION WITH THE
BIENNIAL CONFERENCE OF THE SOCIÉTÉ
FRANÇAISE DE GÉNIE BIOLOGIQUE ET
MÉDICAL (SFGB, 22 August 2007 (2007-08-22),
pages 6247-6250, XP031337662, ISBN:
978-1-4244-0787-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 921 990 B1

**Description**

FIELD OF THE INVENTION

The invention relates to a computing device-implemented method and apparatus for unsupervised segmentation of microscopic color image of unstained specimen and digital staining of segmented histological structures. Segmented histological structures and digitally stained image are displayed in order to diagnose a disease. Some benefits of application of the invention, when compared against existing staining techniques, are: (*i*) shortening of slide preparation process; (*ii*) reduction of variation in diagnosis between histologist; (*iii*) total elimination of adding chemical effects on a specimen; (*iv*) elimination of morphological changes of a specimen e.g. shrinkage; (v) simplification of histological and intra-surgical tissue analysis; (*vi*) being significantly cheaper; (*vii*) harmless to the user because toxic chemical stains are not used; (*viii*) discrimination of several types of histological structures present in the specimen; (*ix*) using the same specimen for more than one analysis.

BACKGROUND OF THE INVETION

Various stains and tags can be attached to biological tissues to enhance contrast of tissue components and thereby improve visibility. The presence, concentration, localization and distribution of biological molecules (such as nucleic acids, proteins or lipids for example) or different portions and structures of the tissue can be determined by selecting a specific combination of chemical fixatives and stains. Visualization of the histological structures in a biological tissue sample is a basic procedure undertaken by pathologist in order to establish diagnosis of the disease that might have afflicted a patient, for example, kidney disease, liver disease, and the like. Staining, however, involves few hours of preprocessing of the specimen, during that some chemical effects can be added to the nature of the cells or tissues, causing their shrinkage and/or other type of morphological changes. For example, in studying effects of DNA damage to cell viability, fluorescent probes cannot be employed to stain the cell nuclei because the viability of the cultures must not be comprised. When studying the inhibitory effects of compounds designed to block the replication of cancerous cells, fluorescent dyes, due to their toxicity, cannot be used to mark their nuclei. Subcellular localization of the genetically encoded proteins imposes constraints on cell recognition method. That is necessary to draw conclusion about a protein's function: staining of the cell is not allowed in order to preserve the quality of the specimen and not influence the result of an investigation. Once a biological tissue is stained with a particular stain or tag to visualize one tissue component, the same tissue generally cannot be stained again with another dye or tag to visualize another tissue component.

When staining is not used, contrast between the histological structures present in the image will be poor, that is they appear colorless when viewed under a light microscope. That is because their spectral profiles are very similar. Thus, it is hard to discriminate (visualize) histological structures in the image of unstained specimen. Digital technology has been developing recently to digitally "stain" images. Digital staining of an image is understood as the process of digitally converting the original image into an image with visual characteristics mimicking those that would be observed if the tissue was stained with a dye conventionally. The advantages of digital staining are multifold. For example, digital staining provides a quantitative result, which could aid diagnosis and reduce the hands-on time of a trained histopathologist as well as reduce intra-histologist variation in diagnosis. It offers the opportunity to develop a variety of digital staining procedures and has the potential to be significantly cheaper than existing chemical staining techniques. Moreover, digital staining does not destroy the biological sample and therefore the same sample could be analyzed by multiple digital staining protocols. Finally, the digital staining process does not involve toxic chemical stains, and is, therefore, intrinsically harmless to the user.

Image segmentation refers to the partitioning of an image into sets of pixels (segments) corresponding to distinct objects. It is understood within the scope of the present invention that the expression distinct objects refers to distinct histological structures present in the image of unstained specimen. Segmentation results are often displayed by a region coloring, i.e. assigning colors to the pixels such that different colors correspond to different objects. This results in compact representation of an image in terms of its useful parts. It is important to distinguish between single- and multi-channel images. In the former case, segmentation is performed by detection of changes of intensity or texture by thresholding some kind of spatial derivative of an image: D. Marr and E. Hildredth, "Theory of Edge Detection," Proc. Royal Soc London Ser B Biol Sci 1980, 207: 187-217. This also can be applied to change in color. More sophisticated versions include relaxation labeling, nonlinear diffusion, Markov random fields, which sometimes are optimized using graph cuts, active contours and/or level sets. These methods often yield analog values rather than binary and that sometimes can be interpreted as a probability that pixel belongs to a specific object. Segmented image by binary outcome is than obtained by thresholding analog values. The threshold value has to be predefined and that is not easy to do in practice because of the varying level of intensity. In addition to a threshold, outlined algorithms may need other parameters to be defined *a priori.* Thus, they are called supervised image segmentation methods.

The present invention is related to an algorithm for unsupervised segmentation of microscopic color (RGB) image of unstained specimen in histopathology and digital staining of segmented histological structures. Thereby, unsupervised segmentation is performed by novel algorithm for underdetermined blind separation of binary {0, 1} sources, whereas in the present invention the term sources means histological structures. Underdetermined blind separation implies that number of histological structures present in the image is greater than number of channels and that is 3 for color (RGB) image. The color image is represented by a linear mixture model comprised of a product of mixing matrix, the columns of which stand for spectral profiles of the histological structures, and binary source matrix. It is further assumed that color image has good spatial resolution and that at each pixel only one histological structure is present. Thus, source matrix indicates whether at some spatial (pixel) location specific histological structure is present or not. When this model represents microscopic color image of unstained specimen, spectral profiles (columns of the mixing matrix) of the histological structures present in the image are highly collinear. That is, the image has a poor spectral resolution and histological structures are hard to distinguish. That is why image segmentation algorithms, including existing blind source separation methods, yield inaccurate results in segmentation of color microscopic image of unstained specimen. To this end, the present invention performs pixel-wise nonlinear mapping of the color image of unstained specimen by using empirical kernel map onto high-dimensional space. Mapped image is represented by the linear mixture model comprised of the same binary sources but with new mixing matrix comprised of high-dimensional mixing vectors that are less collinear than in the case of original image. Thus, spectral discrimination between the histological structures present in the image is improved by nonlinear mapping. Image segmentation is executed by applying sparseness constrained nonnegative matrix factorization (NMF) algorithm to a mapped image. Thereby, NMF algorithm regularized by the $\ell_0$ quasi-norm of the source matrix (the $\ell_0$ quasi-norm counts number of non-zero coefficients of the source matrix) is the method of choice. That is because when source amplitudes belong to {0, 1}, regularization that emulates indicator function, such as $\ell_0$ quasi-norm, is an appropriate choice.

Papers cited below present methods for blind separation of finite-alphabet and binary sources from linear or nonlinear mixtures. They are indirectly related to the subject of the present invention: underdetermined blind separation of binary {0, 1} sources from nonlinear mixtures. That is because binary sources are special case of finite-alphabet sources but also because in case of binary sources nonlinear mixture model is reduced to the linear one. Essential distinctions between subject of the present invention and methods presented in the papers cited below are: (*i*) while in cited references binary sources have values {-1, 1} in present invention they have values {0, 1} and that is more appropriate to model image segmentation; (*ii*) the method of the present invention is using empirical kernel map (EKM)-based nonlinear mapping of the linear mixture model composed of binary sources while cited papers do not perform any kind of nonlinear mapping/transformation of the linear mixture model. Since binary sources have values in {0, 1}, EKM-based nonlinear mapping preserves the sources in mapped space. At the same time, nonlinear mapping increases number of mixtures which makes possible to separate sources from ill-posed linear mixture model and that occurs when histological structures, present in the color microscopic image, have similar spectral profiles, that is when spectral resolution between them is poor. Competing methods for blind separation of binary and/or finite-alphabet sources are presented in: K. Diamantaras, T. Papadimitrou, G. Vranou, "Blind separation of multiple binary sources from one nonlinear mixture," Proc. IEEE Int. Conf. Acoust. Speech and Sig. Proc. (ICASSP-2011), pp.2108-2111, 2011; K. Diamantaras, "Blind separation of multiple binary sources using a single linear mixture," in Proc. IEEE Int. Conf. Acoustic, Speech and Signal Processing (ICASSP-2000), vol.V, pp. 2889-2892, Istanbul, Turkey, June, 2000; K. I. Diamantaras, "A Clustering Approach for Blind Separation of Multiple Finite Alphabet Sequences from a Single Linear Mixture," Signal Processing, vol. 86, Issue 4, pp. 877-891, Elsevier, April, 2006; K. I. Diamantaras, T. Papadimitriou, "Separating two binary sources from a single nonlinear mixture," in Proc. Int. Conf. Acoustics, Speech and Signal Processing (ICASSP 2010), pp. 1946-1949, Dallas, TX, March 14-19, 2010; K. I. Diamantaras, T. Papadimitriou, "Blind separation of three binary sources from one nonlinear mixture," in Proc. 2010 Int. Workshop on Machine Learning for Signal Processing (MLSP-2010), pp.301-306, Kittila, Finland, August 29-September 1, 2010; M. Castella, "Inversion of Polynomial Systems and Separation of Nonlinear Mixtures of Finite-Alphabet Sources" IEEE Trans. on Sig. Proc., vol. 56, No. 8, pp. 3905-3917, August, 2008; Y. Li, A. Cichocki, L. Zhang, "Blind separation and extraction of binary sources" IEICE Trans. Fundamentals, vol. E86-A, no. 3, pp. 580-589, March, 2003; K. I. Diamantaras, T. Papadimitriou, "Blind deconvolution of multi-input single-output systems with binary sources," IEEE Trans. Signal Process., vol. 54, no. 10, pp. 3720-3731, Oct. 2006; K. I. Diamantaras, E. Chassioti, "Blind separation of n binary sources from one observation: A deterministic approach," in Proc. Int. Conf. Independent Component Analysis and Signal Separation (ICA), Helsinki, Finland, June, 2000.

Papers and patents listed below present methods for blind separation of sources from nonlinearly mapped linear mixture model (LMM). The essential differences between subject of the present invention and cited methods are: (i) nonlinear mapping in the present invention is empirical kernel map (EKM) while cited methods use explicit feature map (EFM). While EFM maps LMM in the finite dimensional space, EKM maps LMM in the low-dimensional subspace approximation of the infinite dimensional space. This difference is crucial for discrimination of histological structures with very similar spectral profiles and that is the case with histological structures present in the color microscopic image of the unstained specimen in histopathology; (*ii*) nonnegative matrix factorization (NMF) algorithm employed in mapped space by present invention is regularized the by the $\ell_0$ quasi-norm of the source matrix (the $\ell_0$ quasi-norm counts number of non-zero coefficients of the source matrix). That is due to the fact that source amplitudes belong to {0, 1} and regularization that emulates indicator function, such as $\ell_0$ quasi-norm, is a natural choice. As opposed to that, algorithms used by competing methods cited below employ factorization methods that do not take into account binary nature of the source amplitudes. The methods of indirect relevance to the subject of the present invention are published in: I. Kopriva, M. Hadžija, M. Popović-Hadžija, M. Korolija, A. Cichocki, "Rational Variety Mapping for Contrast-Enhanced Nonlinear Unsupervised Segmentation of Multispectral Images of Unstained Specimen," *The American Journal of Pathology,* vol. 179, No. 2, pp. 547-553, 2011; I. Kopriva, A. Peršin, "Unsupervised decomposition of low-intensity low-dimensional multi-spectral fluorescent images for tumour demarcation," *Medical Image Analysis* 13, 507-518, 2009; Q. Du, I. Kopriva, and H.Szu, "Independent Component Analysis for Classifying Multispectral Images with Dimensionality Limitation," International Journal of Information Acquisition, vol. 1, no. 3, pp.201-216, September 2004; I. Kopriva, X. Chen, Y. Jao, "Nonlinear Band Expansion and Nonnegative Matrix Underapproximation for Unsupervised Segmentation of a Liver from a Multi-phase CT image," SPIE Medical Imaging-Image Processing, Orlando, FL, USA, February 12-17, Proc. SPIE Vol. 7962,79623A-1-79623A-8, Dawant, Benoit, Haynor, David, editors, 2011; I. Kopriva, A. Cichocki, "Nonlinear Band Expansion and 3D Nonnegative Tensor Factorization for Blind Decomposition of Magnetic Resonance Image of the Brain," in Proc. of 9th Int. Conf. on Latent Variable Analysis and Signal Separation, Lecture Notes Computer Science 6365, pp. 490-497, V. Vigneron (editor), September 27-30, 2010, Saint Malo, France; Ouyang, Y. C., Chen, H. M. , Chai, J. W., Chen, C. C. C. , Poon, S. K., Yang, C. W. , Lee, S. K. , Chang, C. I., "Band Expansion-Based Over-Complete Independent Component Analysis for Multispectral Processing of Magnetic Resonance Image," IEEE Trans. Biomed. Eng. 55, 1666-1677, 2008.

There are patents and patent applications where methods for enhancement of images of stained samples are developed. They are mostly not commented here because the subject of the present invention relates to a method for digital processing of the color microscopic image of unstained samples, and are thus not of immediate relevance to the present invention

US Patent 8,532,376 "Signal processing system and computer readable medium for recording signal processing program," relates to endoscope based system and image analysis method that looks for a presence of an object with predefined spectral response. As opposed to that, the method of present invention performs unsupervised segmentation of microscopic color image of unstained specimen into constituent objects without using any prior information.

The US Patent Application 20130071002 "System and method for support of medical diagnosis" relates to automated evaluation of a conventionally prepared sample by a standard dye such as hemotoxylin-eosin by preparing digitally stained sample as a response to staining with a disease specific dye in order to estimate whether staining with disease specific dye is necessary. As opposed to that, the present invention relates to a method for digital staining of the microscopic color image of an unstained specimen, that is no staining with either standard or disease specific dye is required.

The US Patent Application 20120269417 "Computer-aided staining of multispectral images," relates to evaluation and analysis of histological structures and, in particular, to revealing the morphology of these structures by digitally emulating the effects produced by staining the histological structures with dyes. Specifically, this patent application is proposing a method for enhancement of spectral signals of the multispectral image of unstained specimen in order to discriminate objects with similar spectral attributes. That is also a goal of the present invention. However, method proposed in commented patent application is achieving this goal by using multispectral image of unstained specimen and training image related to target chemical stain. As opposed to that, the method of the present invention is fully unsupervised, that is it only uses as its input microscopic color (RGB) image of unstained specimen and does not include any human involvement.

The US Patent Application 20110026803 "Methods and systems for digitally enhancing an image of a stained material," proposed a method for digital adjustment of intensities of an image of the specimen stained with different dyes. As opposed to that the method of the present invention executes digital staining on the microscopic color (RGB) image of unstained specimen.

The US Patent Application 20070016081 "Chroma-photon staining" proposed a digital staining method that modifies a chrominance and luminance components of an image in order to create an image of the particular regions which appear to look as to pre-stained tissue sample. It is not clear from description of this invention how proposed method performs in discriminating objects with similar spectral attributes. As opposed to that, the method of the present invention is developed for discrimination of objects with similar spectral attributes and that is the case with the microscopic color (RGB) image of unstained specimen.

Patent application WO2011078980 "A method for performing a blood count and determining the morphology of a blood cell," presents a method that counts blood cells in a sample of whole blood. Thereby, sample can be stained or unstained. As opposed to the this patent, application of the subject of the present invention relates to an unsupervised image segmentation method that can be applied to color image of arbitrary unstained sample and in order to discriminate between different histological structures (objects) that are present in the image.

Patent application CN101667299 "Method for staining digital image," presents a method for staining a digital image. For this purpose disclosed method selects color image with the content similar to the black and white image as a color source. As opposed to that, the method of the present invention is fully unsupervised. That is the method of the present invention is using only microscopic color image of unstained specimen to perform segmentation and digitally stain segmented histological structures.

Patent application US2010111382 "Method of supporting the differentiation of corneocytes," discloses a method for digital staining of image of unstained sample of corneocytes in order to differentiate skin conditions. The image of unstained corneocytes is converted into pseudocolor image by means of conversion table. As opposed to that, the method of the present invention employs novel blind source separation algorithm for unsupervised segmentation of color image of arbitrary unstained specimen and optionally digitally coloring segmented histological structures and displaying them as pseudocolor image.

Patent application US2013317369 "Devices, Systems and Methods for Virtual Staining," discloses a method for virtual staining of unstained biological tissue. Thereby, image of unstained tissue can be generated by a plurality of detectors (sensors). Image of unstained tissue is transformed digitally such that output image mimics response of tissue to staining by specific stain, dye or group of them. The virtual staining transforms, that are stored in computer system memory, have to be learned. That is achieved by analyzing image of unstained tissue as well as by analyzing image of tissues stained by predefined dyes (stains). As opposed to described invention, the method of the present invention is fully unsupervised, that is it uses only microscopic color image of unstained specimen to segment it into different histological structures and, afterwards, optionally digitally color segmented structures according to predefined color map and display them as a synthetic color (RGB) image.

Patent application US20110228072 "Generation of a multicolor image of an unstained biological specimen," discloses a method for digital (virtual) staining of an image of unstained biological specimen. That is achieved by generating at least two different chemical substances of the unstained specimen and that is used to generate multicolor image of unstained specimen. In another aspect of the invention comprises an optical system for exposing to ultraviolet light at different frequencies unstained biological sample and measuring for the intensity of transmitted ultraviolet light for various regions of the specimen. Measured intensity images are used by computer program to generate multicolor image of unstained specimen. As opposed to described invention, the method of the present invention records only one color (RGB) image of unstained biological specimen. An algorithm stored in computer program is used to segment the image into different histological structures and, optionally, generate synthetic multicolor image.

Patent application US20110134233 "Imaging system and method for enhancing microscopic images of unstained cells," discloses a method for enhancing microscopic image of unstained cells. That is achieved by an imaging system configured to acquire image of unstained cells at different focal planes. Processing of these images yields enhanced image of unstained cell. As opposed to described invention, the method of the present invention records only one color

(RGB) microscopic image of unstained biological specimen. An algorithm stored in computer program is used to segment the image into different histological structures and, optionally, generate synthetic multicolor image.

Patent application US2011005817 "Marker-free chromosome screening," discloses an invention based upon spectral imaging technique which depending on the wavelength produces images of morphological and chemical constituents of a chromosome by means of its interference properties. As opposed to described invention, the method of the present invention records color (RGB) microscopic image of unstained biological specimen that can be composed of various histological structures. That offers an advantage over invention disclosed in US20110058177 because the RGB imaging system is more standard and simpler than multispectral imaging system.

Patent application US20080032325 "Phase subtraction cell counting method," discloses an invention for counting cells in living tissue. That is achieved by obtaining microscopic image of unstained tissue and subtraction of ellipses-based cell models from acquired image. Thus, disclosed image processing method is highly specific. As opposed to described invention, the method of the present invention records color (RGB) microscopic image of unstained biological specimen that can be composed of arbitrary histological structures.

Patent application US20070109874 "Time-lapse cell cycle analysis of unstained nuclei," discloses method for automatic tracking of cell progress over time. For this purpose level set algorithm is used to segment sequence of images, whereas specimen can be stained or unstained. The level set is a supervised image segmentation method that requires user intervention/information during segmentation process. As opposed to described invention, the method of the present invention performs unsupervised segmentation of a single color (RGB) microscopic image of unstained biological specimen that can be composed of arbitrary histological structures.

Patent application JPH0225251 "Microscope for Microsurgery" discloses an apparatus (microscope system) capable of processing an unstained specimen by arranging special construction of a microscope. As opposed to described invention, the method of the present invention discriminates histological structures in color microscopic image of unstained specimen by means of algorithm that performs unsupervised segmentation of the image.

Patent application CA1036385 "Biological cell analyzing system," discloses a method for automatic categorization of unstained biological cells as normal and non-normal. Thereby, the cells are made to flow through a transparent tube and are scanned with a mixture of ultraviolet and visible light. Categorization is achieved by subtracting visible light signal from ultraviolet light signal. As opposed to described invention, the method of the present invention discriminates arbitrary histological structures in color microscopic image of unstained specimen by means of algorithm that performs unsupervised segmentation of the image.

Patent application US20120147002 "Virtual Cellular Staining," discloses a method for virtual staining of cellular structures in order to prevent or minimize crosstalk among emitted colors in an imaging experiment that involves multiplex staining. Thereby, virtual cellular staining involves displaying one or more structures of an exemplary cell on a display of an electronic device and allowing a user to change the individual colors of the one or more structures of the cell. It is thus assumed in the embodiment of disclosed invention that cellular structures present in the image are distinguishable to the user. As opposed to described invention, the method of the present invention discriminates arbitrary histological structures in color microscopic image of unstained specimen by means of algorithm that performs unsupervised segmentation of the image. Since the specimen is unstained, histological structures present in the image are hardly visible to the user.

Accordingly, it is the aim of the present invention to provide a method and system for unsupervised segmentation of color (RGB) microscopic image of unstained specimen and digital staining of segmented (discriminated) histological structures present in the specimen.

SUMMARY OF THE INVENTION

This aim is achieved by means of blind separation of non-overlapping binary {0, 1} sources from smaller number of linear mixtures. Thereby, sources represent presence or absence of the histological structure (histological structure can refer to cell, nuclei, tissue types and the like) at particular spatial location (pixel element) in the image and mixtures represent image intensities recorded at particular color (wavelength) such as red, green and blue (RGB). The invention is composed of empirical kernel map (EKM)-based nonlinear mapping of recorded microscopic color (RGB) image of unstained specimen onto high-dimensional space and $\ell_0$ quasi-norm constrained nonnegative matrix factorization (NMF) of mapped image, characterised in that said underdetermined blind separation of non-overlapping binary {0, 1} sources comprises the following steps:

- recording and storing the microscopic color (RGB) image of unstained specimen **X**, where $\mathbf{X} \in \mathbb{R}_{0+}^{3 \times T}$ stands for nonnegative data matrix comprised of 3 rows that correspond to vectorized gray scale images at red, green and blue color channels and $\left\{ \mathbf{x}_t \right\}_{t=1}^{T}$ columns representing intensity values of $T$ pixels;

- scaling the image data matrix by maximal element of **X**, $x_{max}$:

$$\mathbf{X}=\mathbf{X}/\mathrm{x_{max}} \qquad\qquad [\mathrm{I}]$$

- representing image data matrix **X** by linear mixture model:

$$\mathbf{X}=\mathbf{AS} \qquad\qquad [\mathrm{II}]$$

where $\mathbf{A} \in \mathbb{R}_{0+}^{3 \times M}$ stands for nonnegative mixture matrix comprised of $M$ column vectors $\left\{ \mathbf{a}_m \right\}_{m=1}^{M}$ that stand for spectral profiles of $M$ histological structures present in the image X; S stands for $M \times T$ binary source matrix comprised of {0, 1} values such that element $\left\{ s_{mt} \in \{0,1\} \right\}_{m,t=1}^{M,T}$ indicates presence (1) or absence (0) of the histological structure $m$ at a pixel location $t$.

- using empirical kernel map for nonlinear mapping of **X** in [II] onto reproducible kernel Hilbert space $\Psi\left(\mathbf{X}\right) \in \mathbb{R}_{0+}^{D \times T}$:

$$\Psi\left(\mathbf{X}\right) = \begin{bmatrix} \kappa\left(\mathbf{x}_1, \mathbf{v}_1\right) & ... & \kappa\left(\mathbf{x}_T, \mathbf{v}_1\right) \\ ... & ... & ... \\ \kappa\left(\mathbf{x}_1, \mathbf{v}_D\right) & ... & \kappa\left(\mathbf{x}_T, \mathbf{v}_D\right) \end{bmatrix} \qquad \text{[III]}$$

where $k(\mathbf{x}_t, \mathbf{v}_d)$, $t$=1,..., $T$ and $d$=1, ..., $D$ stands for positive symmetric kernel function and $\mathbf{v}_d$, $d$=1, ..., $D$ stand for basis vectors that approximately span the same space as pixels vectors: $\mathbf{x}_t$, $t$=1, ..., $T$ such that $D \gg 3$.

- representing mapped matrix $\Psi$ (**X**) by linear mixture model [IV]:

$$\Psi\left(\mathbf{X}\right) = \mathbf{BS} \qquad \text{[IV]}$$

such that S is the same binary source matrix as in [II], while $\mathbf{B} \in \mathbb{R}_{0+}^{D \times M}$ is mixing matrix in mapped space such that column vectors $\left\{\mathbf{b}_m\right\}_{m=1}^{M}$ are mutually significantly less correlated than column vectors $\left\{\mathbf{a}_m\right\}_{m=1}^{M}$ in [II]. Thus, it will be easier to discriminate histological structures present in the image **X** by factorizing [IV] than by factorizing [II];

- applying sparseness and nonnegativity constrained matrix factorization (sNMF) algorithm to [IV], whereas sparseness constraint is based on indicator function of **S** such as $\ell_0$ quasi-norm of **S**, to obtain estimates of the presence/absence of histological structures $\left\{\mathbf{s}_m\right\}_{m=1}^{M}$:

$$\left\{\hat{\mathbf{s}}_m\right\}_{m=1}^{M} = sNMF\left(\Psi\left(\mathbf{X}\right)\right) \qquad \text{[V]}$$

where as in [II] $M$ denotes number of histological structures present in the image **X**;

- displaying segmented histological structures $\left\{\hat{\mathbf{s}}_m\right\}_{m=1}^{M}$ as binary (black and white) maps;
- alternatively, coloring each segmented histological structure with some predefined color according to:

$$\mathbf{Y} = \mathbf{C}\hat{\mathbf{S}} \qquad \text{[VI]}$$

where $\left\{\mathbf{c}_m\right\}_{m=1}^{M}$ stand for some predefined vectors in RGB-color space and displaying segmented histological structures as synthetic color (RGB) image **Y**.

Further, this aim is achieved by a system for unsupervised image segmentation by means of underdetermined blind separation of nonnegative binary sources comprising: light microscope 101 and color (RGB) camera 102 for recording image **X** of unstained specimen, an input storing device/medium 103 for storing the image **X,** a computing device 104 wherein code is implemented or carried out for executing a method according to anyone of the claims **1** to **12** based on image **X** stored in/on the input storing device/medium 103, an output storing and displaying device or medium 105 for storing and displaying the result of the method carried out by the processor.

Preferably, positive symmetric kernel functions in [III] is selected as Gaussian kernel:

$$\kappa\left(\mathbf{x}_t, \mathbf{v}_d\right) = \exp\left(-\left\|\mathbf{x}_t - \mathbf{v}_d\right\|^2 / \sigma^2\right).$$

Conveniently, $\sigma^2 \approx 0.1$.

Preferably, subspace dimension in empirical kernel map induced space in [III] is $D \approx 150$.

Preferably, number of histological structures present $M$ in [II], [III] and [V] is selected as $M \in \{4, 5, 6\}$.

Preferably, $sNMF$ algorithm in [V] is nonnegative matrix factorization algorithm constrained with $\ell_0$ - quasi-norm of S: R. Peharz, F. Pernkopf, "Sparse nonnegative matrix factorization with $\ell 0$ constraints," Neurocomputing, vol. 80, pp. 38-46, 2012.

According to a further special embodiment, a method of the present invention is applied to discrimination of histological structures present in the color microscopic image of unstained specimen.

The present invention has been described in terms of specific embodiments incorporating details to facilitate the understanding of principles of construction and operation of the invention. Such reference herein to specific embodiments and details thereof is not intended to limit the scope of the claims appended hereto. It will be readily apparent to one skilled in the art that other various modifications may be made in the embodiment chosen for illustration without departing from the spirit and scope of the invention as defined by the claims.

As used herein, the terms "stain" and "staining" are broad terms and can include without limitation staining with a dye or a stain, immunohistochemical staining, aptamer staining, tagging, chemical staining, antibody staining, or any other alteration to a tissue sample.

As used herein, the terms "sample," "tissue sample," "biological sample," and "specimen" may be used interchangeably, and the foregoing terms comprise without limitation tissue samples, tissue specimen, bulk tissue, surgical site, biopsy, bacteria, cell or cell components. A sample can be analyzed in vivo or in vitro.

Furthermore, the present invention provides a computer-readable medium having computer executable instructions stored thereon, which, when executed by computer will cause the computer to carry out a method of the present invention. Common forms of computer-readable media include, for example, a floppy disk, a flexible disk, a hard disk, a magnetic tape, or any other magnetic medium, a CD-ROM, any other optical medium, a punch card, a papertape, any other physical medium with patterns of holes, a RAM, a PROM, an EPROM, a FLASH-EPROM, any other memory chip or cartridge, or any other tangible and non-transitory present or future medium from which a computer can read.

In a preferred embodiment of the system, the output storing and displaying device is a printer, plotter or display monitor and the output storing medium is a memory based device that is readable by computer.

The novelty of proposed method for unsupervised segmentation of color microscopic image of unstained specimen and digital staining of segmented histological structures in relation to state-of-the-art is in empirical kernel map based nonlinear mapping of the color image of unstained specimen onto high-dimensional space. This mapping embeds spectral profiles of the histological structures from 3-dimensional space to space with much higher dimensionality (for example 150-dimensional space). That enables discrimination between histological structures with very similar spectral profiles and that occurs when color microscopic image of unstained specimen is recorded. Therefore, factorization of mapped image constrained by nonnegativity and indicator function of the sources, such as $\ell_0$ quasi-norm of the sources, will discriminate/segment histological structures even though, due to spectral similarity, they are hardly visible in the image of unstained specimen.

BRIEF DESCRIPTION OF THE DRAWINGS

A more detailed description of the invention will be given with references to the following figures, in which:

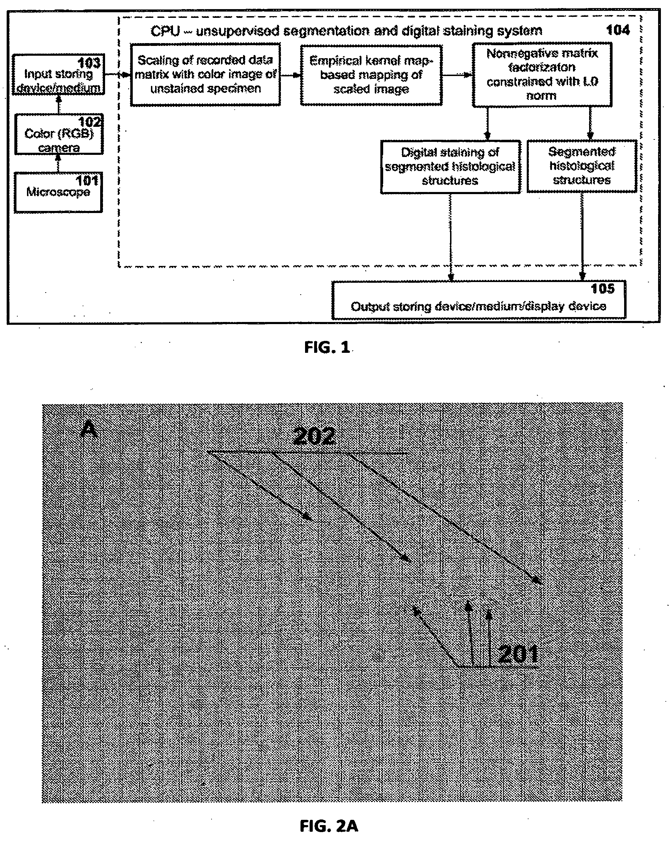

FIG. **1** schematically illustrates a block diagram of a computing device for unsupervised segmentation of color microscopic image of unstained specimen and digital staining of segmented histological structures according to the embodiment of the present invention;

FIGS. **2A** to **2E** show respectively: gray scale version of color microscopic image of unstained specimen of human liver with diagnoses of obesity with marked blood vessels 201 and vacuoles 202; images of segmented histological structures: blood vessels 203 and vacuoles 204; gray scale version of color image obtained by digital staining of segmented histological structures with marked blood vessels 205 and vacuoles 206; gray scale version of the color image of the same specimen stained subsequently with hemotoxylin-eosin with marked histological structures related to blood vessels 207 and vacuoles 208;

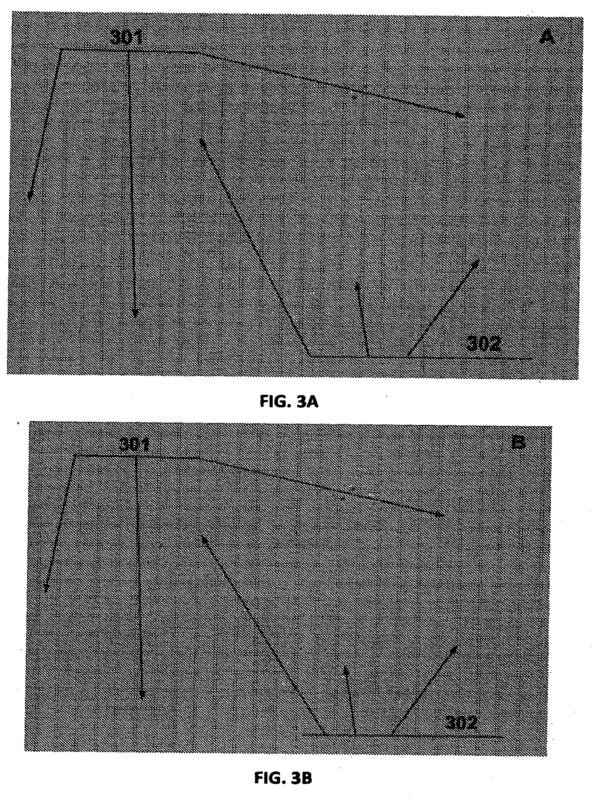

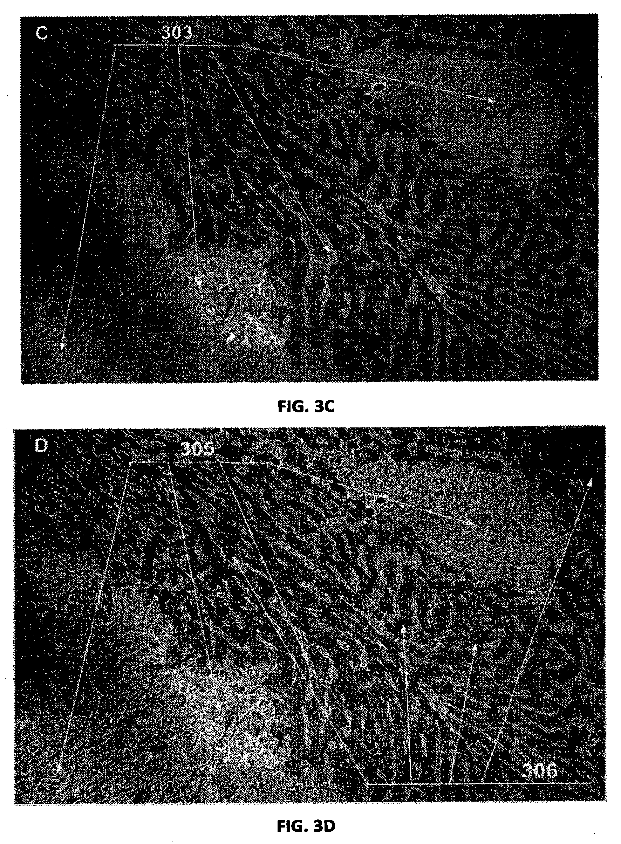

FIGS. **3A** to **3E** show respectively: gray scale version of color microscopic image of unstained specimen of the human liver tissue with marked metastasis 301 arising from primary tumor in pancreas and unaffected liver parenchyma 302; images of segmented histological structures: metastasis 303 and liver parenchyma 304; gray scale version of color image obtained by digital staining of segmented histological structures with marked metastasis 305 and liver parenchyma 306; gray scale version of the color image of the same specimen subsequently stained with hemotoxylin-eosin with marked histological structures related to metastasis 307 and liver parenchyma 308;

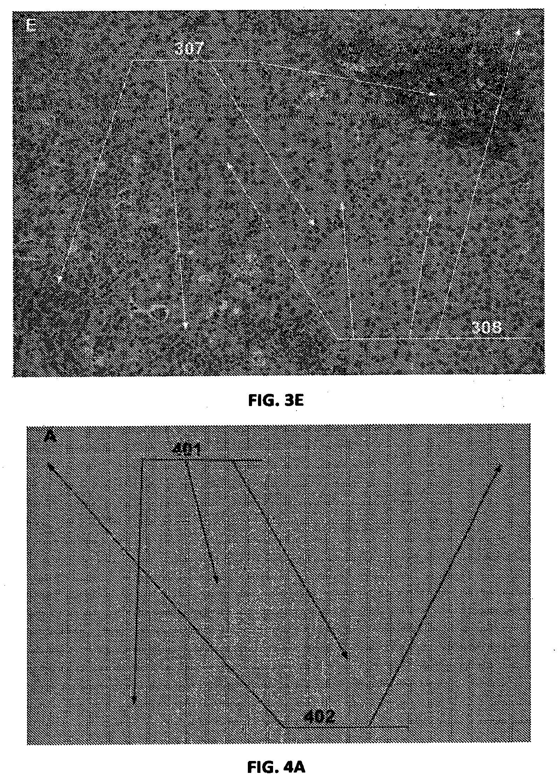

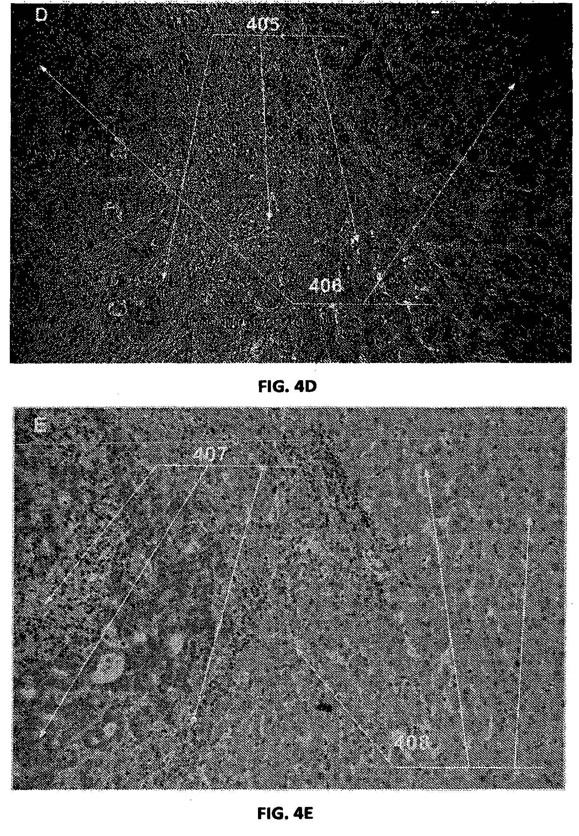

FIGS. **4A** to **4E** show respectively: gray scale version of color microscopic image of unstained specimen of human liver tissue with marked metastasis 401 arising from primary colon carcinoma and unaffected liver parenchyma 402; images of segmented histological structures: metastasis 403 and liver parenchyma 404; gray scale version of color image obtained by digital staining of segmented histological structures with marked metastasis 405 and liver parenchyma 406; gray scale version of the color image of the same specimen subsequently stained with hemotoxylin-eosin with marked histological structures related to metastasis 407 and liver parenchyma -408;

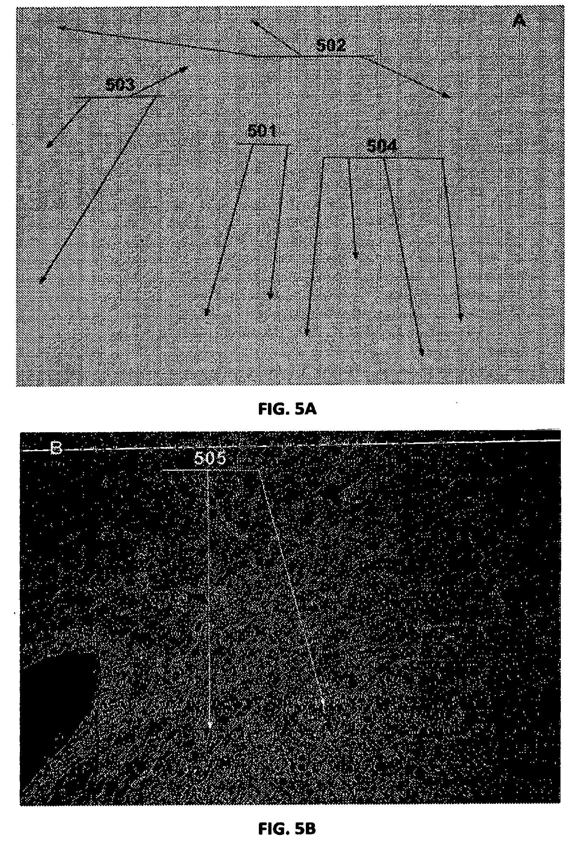

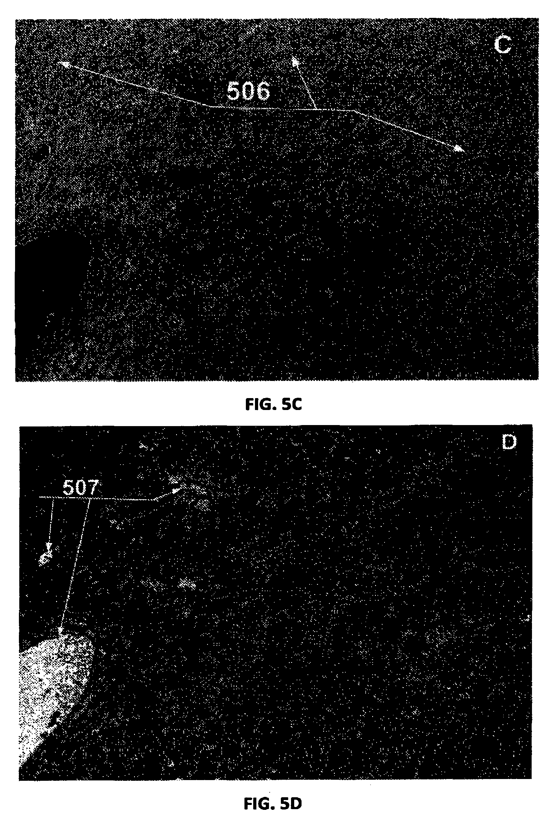

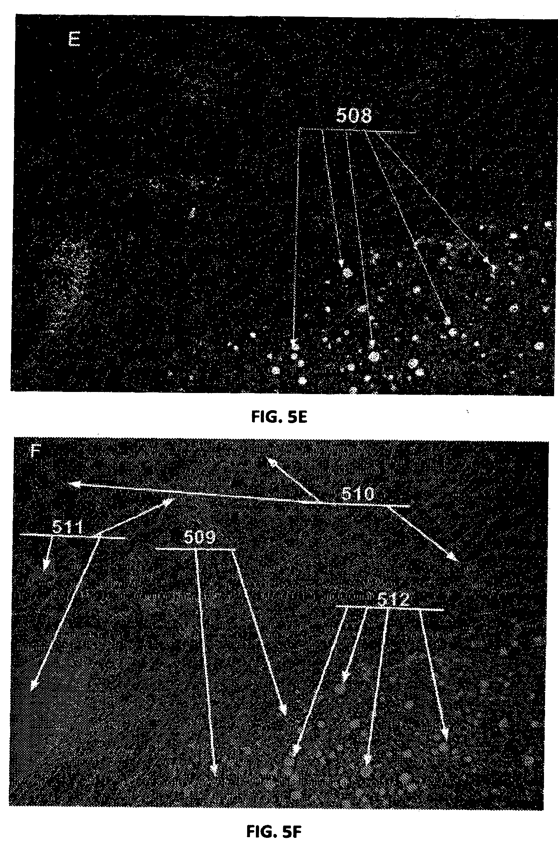

FIGS. **5A** to **5G** show respectively: gray scale version of color microscopic image of unstained liver specimen of a patient with diagnosis of hepatocellular carcinoma with marked tumor 501, liver parenchyma 502, blood vessels 503 and vacuoles 504; images of segmented histological structures: metastasis 505, liver parenchyma 506, blood vessels 507 and vacuoles 508; gray scale version of color image obtained by digital staining of segmented histological structures with marked metastasis 509, liver parenchyma 510, blood vessels 511 and vacuoles 512; gray scale version of the color image of the same specimen stained subsequently with hemotoxylin-eosin with marked histological structures related to tumor 513, liver parenchyma 514, blood vessels 515 and vacuoles 516;

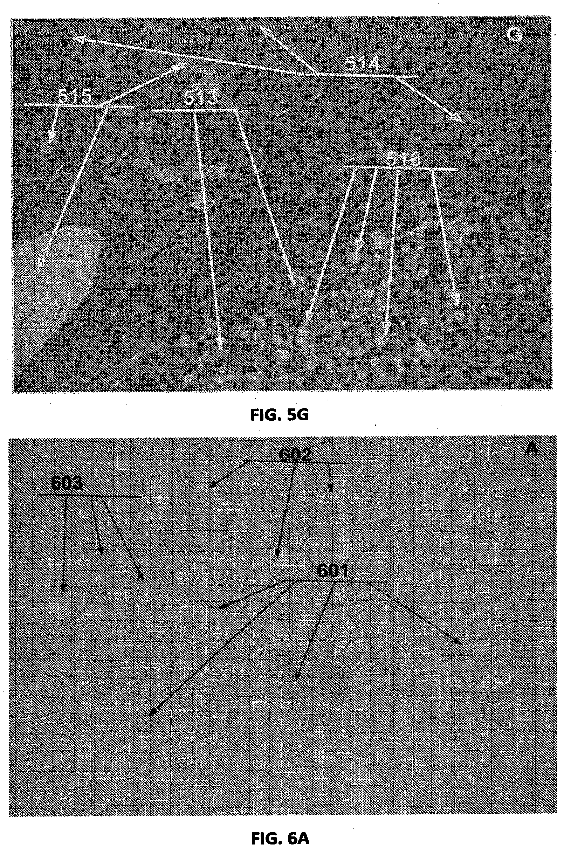

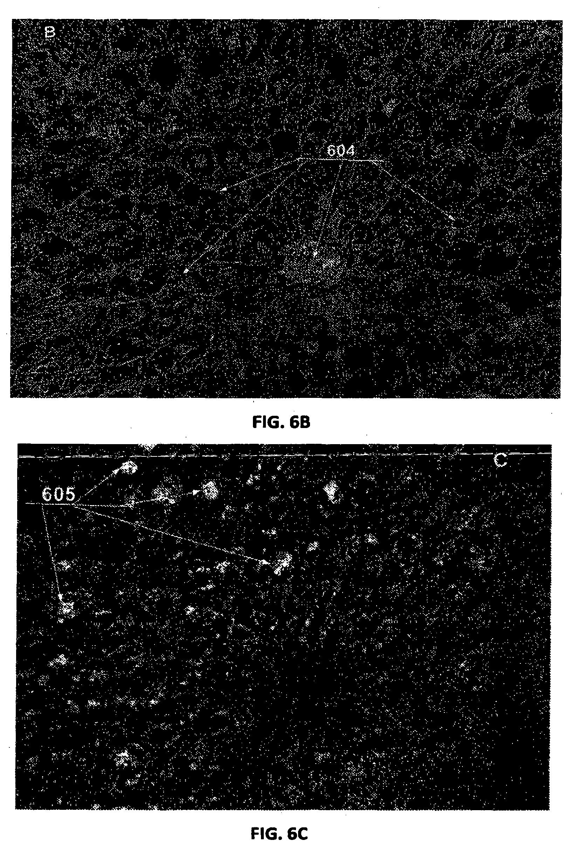

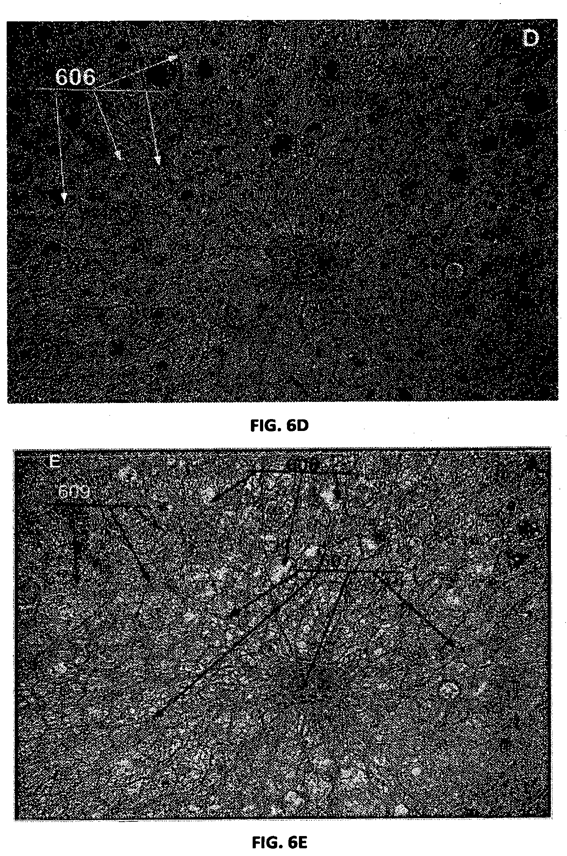

FIGS. **6A** to **6F** show respectively: gray scale version of color microscopic image of unstained specimen of the liver of the NOD mice with marked vena centralis and sinusoids 601, vacuoles 602 and cell membranes 603; images of segmented histological structures: vena centralis and sinusoids 604, vacuoles 605 and cell membranes 606; gray scale version of color image obtained by digital staining of segmented histological structures with marked: vena centralis and sinusoids 607, vacuoles 608 and cell membranes 609; gray scale version of the color image of the same specimen stained subsequently with hemotoxylin-eosin with marked histological structures related to vena centralis and sinusoids 610, vacuoles 611 and cell membranes 612.

DETAILED DESCRIPTION OF THE INVENTION

Embodiments will now be described with reference to the accompanying figures. The terminology used in the description presented herein is not intended to be interpreted in any limited or restrictive manner, simply because it is being utilized in conjunction with a detailed description of certain specific embodiments. Furthermore, embodiments may comprise several novel features, no single one of which is solely responsible for its desirable attribute or which is essential to practicing the embodiments herein described.

A schematic block-diagram of a device for unsupervised segmentation of color microscopic image of unstained specimen and digital staining of segmented histological structures, that is defined by equation [II] and employing methodology of empirical kernel map-based nonlinear mapping and nonnegativity and $\ell_0$-norm constrained matrix factorization, according to an embodiment of the present invention is shown in FIG. **1**. The device consists of: light microscope 101; color (RGB) camera 102 used to acquire color microscopic image of unstained specimen; input storing device 103 used to store acquired image; CPU or computer 104 where algorithm based on empirical kernel map and nonnegativity and $\ell_0$-norm constrained factorization is implemented for unsupervised segmentation of color microscopic image of unstained specimen and digital staining of segmented histological structures; and output storing and/or display device 105 used to store and display segmented histological structures and synthetic color (RGB) image obtained by digital staining of segmented histological structures.

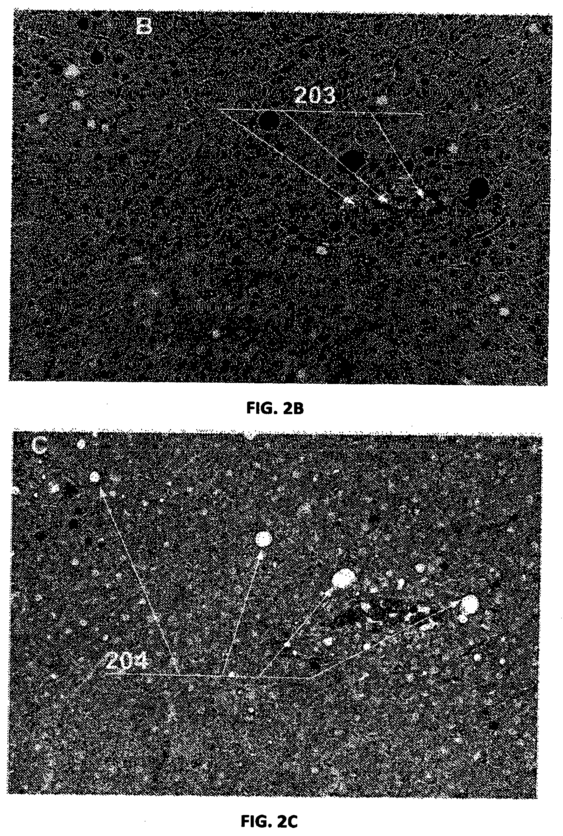

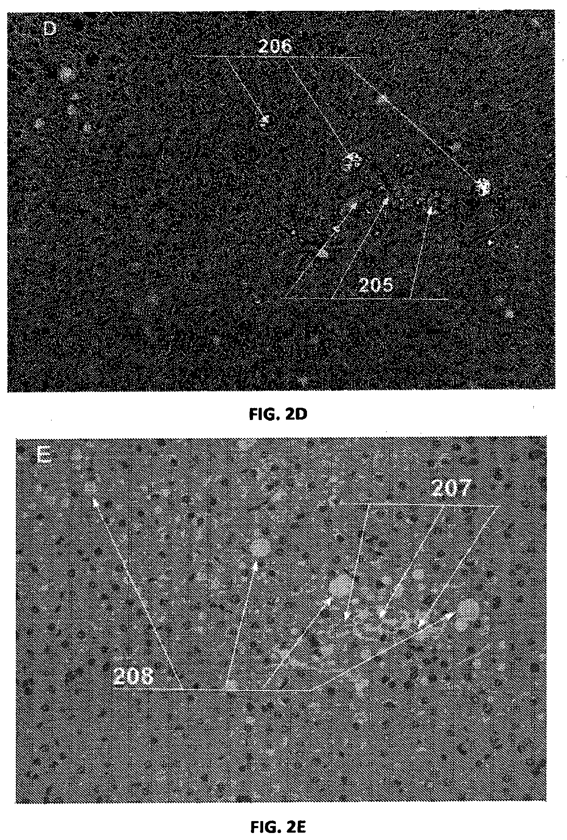

The procedure for processing acquired and stored color microscopic image of unstained specimen, with the aim to perform unsupervised segmentation of the image and digitally stain segmented histological structures, is implemented in the software or firmware in the CPU (104) and, according to an embodiment of the present invention, consists of the following steps: scaling of the acquired image according to equation [I] in order to constrain intensities of the image to be in the range between 0 and 1; scaled image is represented by linear mixture model [II] where source matrix **S** is comprised of binary {0, 1} values such that $s_{mt} \in \{0, 1\}$ indicates whether histological structure $m=1,...,M$ is present at pixel location $t=1,...,T$. Empirical kernel map is used to map scaled image **X** into new matrix $\Psi(\mathbf{X})$ [III], whereas number of mixtures $D$ in [III] is much greater than number of mixtures $N=3$ in color microscopic RGB image **X** in [II]. Because mapping is nonlinear, $D$ mixtures in [III] are linearly independent, that is they are not redundant. Thereby, it is of great importance that vector space spanned by the basis $\left\{\mathbf{v}_d\right\}_{d=1}^{D}$ approximates well the vector space spanned by the empirical set of vectors $\left\{\mathbf{x}_t\right\}_{t=1}^{T}$. To this end, it is possible to find a basis $\left\{\mathbf{v}_d\right\}_{d=1}^{D}$ that spans low-dimensional subspace that approximates well the vector space spanned by the empirical set of vectors $\left\{\mathbf{x}_t\right\}_{t=1}^{T}$. For this purpose, data clustering algorithms or specialized basis selection algorithms can be used. Due to binary {0, 1} character of the sources **S** in [II] empirical kernel map generated matrix $\Psi(\mathbf{X})$ [III] can be represented by linear mixture model [IV] such that source matrix S remains the same as S in [II]. However, because $D>>N$ mixing vectors $\left\{\mathbf{b}_m\right\}_{m=1}^{M}$ in [IV] are less collinear than mixing vectors $\left\{\mathbf{a}_m\right\}_{m=1}^{M}$ in [II]. Since mixing vectors represent spectral profiles of the histological structures present in the image **X** it is easier to segment histological structures from mapped image $\Psi(\mathbf{X})$ in [III]/[IV] than from original image **X** in [II]. To this end, empirical kernel map-generated images in FIGS.2 to FIGS.6 were obtained with $D=150$ and Gaussian kernel $\kappa(\mathbf{x}_t,\mathbf{v}_d) = \exp\{-\|\mathbf{x}_t-\mathbf{v}_d\|^2/\sigma^2\}$ with $\sigma^2 \approx 0.1$. Due to binary {0, 1} character of the sources **S** in [IV] nonnegative matrix

factorization of $\Psi(\mathbf{X})$ in [V] is constrained with $\ell_0$ -norm of $\mathbf{S}$. That yields estimates of the histological structures $\{\hat{\mathbf{s}}_m\}_{m=1}^{M}$ present in the image $\mathbf{X}$. Segmented histological structures are stored and/or displayed at the output device 105. Furthermore, segmented histological structures are digitally stained according to [VI] to obtain synthetic color (RGB) image Y that is stored and/or displayed at the output device 105.

In detail, according to an embodiment of the present invention, procedure for unsupervised segmentation of color microscopic image of unstained specimen and digital staining of segmented histological structures consists of the following steps:

recording color microscopic image of unstained specimen $\mathbf{X}$ by means of light microscope 101 and color (RGB) camera 102,

storing recorded image on the input storing device or medium 103,

scaling stored image according to equation [I] and representing it by linear mixture model [II] as weighted linear combination of the unknown binary matrix $\mathbf{S}$ that indicates presence or absence of the histological structures in the image $\mathbf{X}$,

using empirical kernel map for nonlinear mapping of $\mathbf{X}$ in [II] to obtain new data matrix $\Psi(\mathbf{X})$ in [III] with greater number of linearly independent mixtures than in the original data matrix $\mathbf{X}$ in [II],

applying nonnegative matrix factorization algorithm constrained by $\ell_0$ -norm of $\mathbf{S}$ to mapped matrix $\Psi(\mathbf{X})$ in [V] to segment histological structures present in the image $\mathbf{X}$,

presenting segmented histological structures at the output storing/display device 105

digitally staining segmented histological structures according to [VI] and presenting synthetic color image Y at the output storing/display device 105.

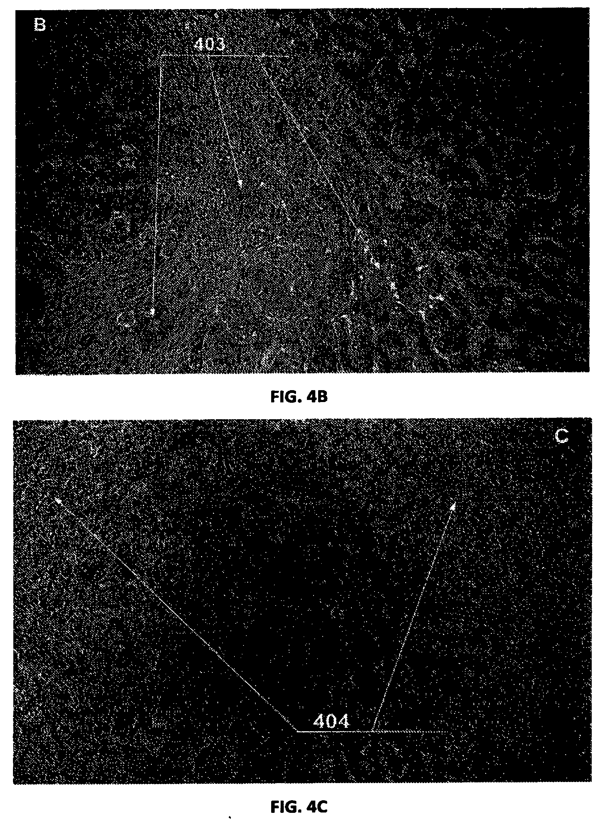

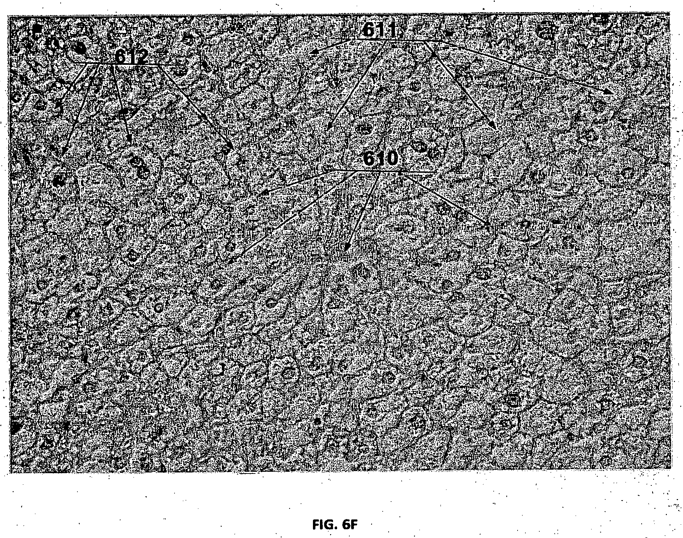

FIGS. 2 to 6 demonstrate unsupervised segmentation of experimental microscopic color images of unstained specimen and digital staining of segmented histological structures on experimental image according to an embodiment of the present invention. FIG. 2A shows gray scale version of color microscopic image of unstained specimen of the human liver with diagnoses of obesity with marked blood vessels 201 and vacuoles 202. Histological structures segmented by means of nonnegative matrix factorization algorithm constrained with $\ell_0$ quasi-norm (NMF_L0) and related to blood vessels 203 and vacuoles 204 are respectively shown in FIG. 2B and FIG. 2C. These structures are digitally stained according to [VI] whereas gray scale version of synthetic color image $\mathbf{Y}$ is shown in FIG. 2D. FIG. 2E shows gray scale version of the color image of the same specimen stained subsequently with hemotoxylin-eosin with marked histological structures related to blood vessels 207 and vacuoles 208. It is seen the correspondence between segmented histological structures shown in FIGS. 2B and 2C and histological structures colored by hemotoxylin-eosin in FIG. 2E. It is, however, important to emphasize that histological structures related to blood vessels and vacuoles were segmented (discriminated) from the image of unstained specimen. FIGS. 3B to 3E show results related to unsupervised segmentation and digital staining of histological structures present in the image of unstained specimen, FIG. 3A, of the human liver with widespread liver metastases from pancreatic cancer. Herein, segmented histological structure that corresponds with the metastasis is shown in FIG. 3B, while segmented histological structure that corresponds with the liver parenchyma is shown in FIG. 3C. It is seen the correspondence between segmented histological structures shown in FIGS. 3B and 3C and histological structures colored by hemotoxylin-eosin in FIG. 3E. It is however important to emphasize that histological structures related to metastasis and liver parenchyma were segmented (discriminated) from the image of unstained specimen of the liver. FIGS. 4B to 4E show results related to unsupervised segmentation and digital staining of histological structures present in the image of unstained specimen, FIG. 4A, of the human liver with widespread liver metastases from colon cancer. Herein, segmented histological structure that corresponds with the metastasis is shown in FIG. 4B, while segmented histological structure that corresponds with the liver parenchyma is shown in FIG. 4C. Again, it is seen the correspondence between segmented histological structures shown in FIGS. 4B and 4C and histological structures colored by standard hemotoxylin-eosin procedure in FIG. 4E. Thereby, histological structures related to metastasis and liver parenchyma were segmented (discriminated) from the image of unstained specimen of the liver. FIGS. 5B to 5G show results related to unsupervised segmentation and digital staining of histological structures present in the image of unstained specimen, FIG. 5A, of liver of patient with diagnosis of hepatocellular carcinoma. Thereby, segmented histological structures related to tumor, liver parenchyma, blood vessels and vacuoles are respectively shown in FIGS. 5B to 5E. It is important to emphasize that histological structures related to blood vessels and vacuoles are very hard to distinguish even on the image stained by hemotoxylin-eosin, i.e. due to very similar spectral profile they are colored equally, see FIG. **5G.** It is also important to emphasize that staining substances (standard H&E staining) cannot discriminate four types of histological structures (vacuoles, blood vessels and liver parenchima). Nevertheless, proposed method was capable to discriminate them from the image of unstained specimen only and digitally stain them in four different colors. FIGS. **6B** to **6F** show results related to unsupervised segmentation and digital staining of histological structures present in unstained specimen of the liver tissue of NOD mice, FIG. **6A.** Segmented structures related to vena centralis and sinusoids, vacuoles filled with lipids and cell membranes are respectively shown in FIGS. **6B** to **6D.** Again, it is seen the correspondence between segmented histological structures shown in FIGS. **6B** to **6D** and histological structures colored by hemotoxylin-eosin in FIG. **6F.** Thereby, histological structures related to metastasis and liver parenchyma were segmented (discriminated) from the image of unstained specimen of the mouse liver.

Empirical kernel map-based nonlinear mapping of the color microscopic image of unstained specimen combined with non-overlapping and binary {0, 1} constraint on the sources (histological structures) is what enables unsupervised segmentation of the image of unstained specimen and digital staining of segmented histological structures. That is distinction with respect to state-of-the-art linear and nonlinear algorithms for blind separation of binary sources that do not employ empirical kernel map based nonlinear mapping of the image. That, however, is of crucial importance for discrimination of spectrally very similar histological structures present in the image of unstained specimen.

The invention relates to a computing device-implemented method and apparatus for unsupervised segmentation of color microscopic image of unstained specimen and digital staining of segmented histological structures. Some benefits of the application of invention in clinical pathology are: (*i*) shortening of slide preparation process; (*ii*) reduction of intra-histologist variation in diagnosis; (*iii*) elimination of the possibility to add chemical effects to a specimen; (*iv*) elimination of the morphological changes (e.g. shrinkage) during staining procedure; (*v*) simplification of histological and intra-surgical tissue analysis; (*vi*) significantly cheaper than existing staining techniques; (*vii*) harmless to the user because toxic chemical stains are not used.

The present invention can be applied to the discrimination and digital staining of the histological structures present in the microscopic color image of the unstained specimen. Elimination of staining brings the following benefits in clinical pathology: (*i*) shortening of slide preparation process; (*ii*) reduction of intra-histologist variation in diagnosis; (*iii*) elimination of adding chemical effects on specimen; (*iv*) elimination of additional morphological changes of a specimen; (*v*) simplification of histological and intra-surgical tissue analysis; (*vi*) significantly cheaper than existing staining techniques; (*vii*) harmless to the user because toxic chemical stains are not used; (*viii*) discrimination of several types of histological structures present in the specimen; (*ix*) allowing the use of the same specimen for more than one analysis.

The features in the foregoing description, in the claims and/or in the accompanying drawings may, both and in the any combination thereof, be material for realizing the invention in diverse form thereof.

## Claims

1. A method for unsupervised segmentation of microscopic color image of unstained specimen and digital staining of segmented histological structures by using empirical kernel map-based nonlinear mapping of recorded microscopic image of unstained specimen onto reproducible kernel Hilbert space, factorization of mapped image constrained' by nonnegativity and $\ell_0$-norm of the binary {0, 1} sources (histological structures) and digital staining of factorized (segmented) histological structures comprising the following steps:

   recording and storing microscopic color image of unstained specimen **X**, where **X** is nonnegative data matrix comprised of *N*=3 rows that correspond to gray scale images recorded at particular wavelengths corresponding to red, green and blue colors and *T* columns that correspond to observations at different spatial (pixel) locations, scaling the image data matrix by maximal element of X, $x_{max}$:

$$\mathbf{X}=\mathbf{X}/x_{max} \qquad\qquad [I]$$

   - representing image data matrix **X** by linear mixture model:

$$\mathbf{X}=\mathbf{AS} \qquad\qquad [II]$$

   where $\mathbf{A} \in \mathbb{R}_{0+}^{3\times M}$ stands for nonnegative mixture matrix comprised of *M* column vectors $\left\{\mathbf{a}_m\right\}_{m=1}^{M}$ that stand for spectral profiles of $M$ histological structures present in the image $\mathbf{X}$; $\mathbf{S}$ stands for $M \times T$ binary source matrix comprised of {0, 1} values such that element $\{s_{mt} \in \{0,1\}\}_{m,t=1}^{M,T}$ indicates presence (1) or absence (0) of the histological structure $m$ at pixel location $t$.

- using empirical kernel map for nonlinear mapping of $\mathbf{X}$ in [II] onto reproducible kernel Hilbert space $\Psi(\mathbf{X}) \in \mathbb{R}_{0+}^{D \times T}$ :

$$\Psi(\mathbf{X}) = \begin{bmatrix} \kappa(\mathbf{x}_1, \mathbf{v}_1) & ... & \kappa(\mathbf{x}_T, \mathbf{v}_1) \\ ... & ... & ... \\ \kappa(\mathbf{x}_1, \mathbf{v}_D) & ... & \kappa(\mathbf{x}_T, \mathbf{v}_D) \end{bmatrix} \qquad\qquad \text{[III]}$$

where $\kappa(\mathbf{x}_t, \mathbf{v}_d)$, $t=1,..., T$ and $d=1, ..., D$ stands for positive symmetric kernel function and $\mathbf{v}_d$, $d=1, ..., D$ stand for basis vectors that approximately span the same space as pixels vectors: $\mathbf{x}_t$, $t=1, ..., T$.

- representing mapped matrix $\Psi(\mathbf{X})$ by linear mixture model [IV]:

$$\Psi(\mathbf{X}) = \mathbf{BS} \qquad\qquad \text{[IV]}$$

such that $\mathbf{S}$ is the same binary source matrix as in [II], while $\mathbf{B} \in \mathbb{R}_{0+}^{D \times M}$ is mixing matrix in mapped space such that column vectors $\{\mathbf{b}_m\}_{m=1}^{M}$ are mutually significantly less correlated than column vectors $\{\mathbf{a}_m\}_{m=1}^{M}$ in [II] that enables discrimination of spectrally similar histological structures present in the image $\mathbf{X}$;

- applying sparseness and nonnegativity constrained matrix factorization (sNMF) algorithm to [IV], whereas sparseness constraint is based on indicator function of $\mathbf{S}$ such as $\ell_0$ quasi-norm of $\mathbf{S}$, to obtain estimates of the presence/absence of histological structures $\{\mathbf{s}_m\}_{m=1}^{M}$ :

$$\{\hat{\mathbf{s}}_m\}_{m=1}^{M} = sNMF(\Psi(\mathbf{X})) \qquad\qquad \text{[V]}$$

where, as in [II], $M$ denotes number of histological structures present in the image $\mathbf{X}$;

- displaying segmented histological structures $\{\hat{\mathbf{s}}_m\}_{m=1}^{M}$ as black and white maps;

- digitally staining (coloring) segmented histological structures $\{\hat{\mathbf{s}}_m\}_{m=1}^{M}$ with predefined colors according to:

$$\mathbf{Y} = \mathbf{C}\hat{\mathbf{S}} \qquad\qquad \text{[VI]}$$

where $\{\mathbf{c}_m\}_{m=1}^{M}$ stand for predefined color vectors in RGB-color space.

- displaying segmented histological structures as synthetic color (RGB) image $\mathbf{Y}$.

2. The method of claim **1**, where in empirical kernel map [III] positive symmetric kernel function is shift invariant kernel: $\kappa(x_t, v_d) = \kappa(x_t\text{-}v_d)$. Preferably, $\kappa(x_t, v_d)$ is Gaussian kernel: $\kappa(x_t, v_d) = \exp(-\|x_t\text{-}v_d\|^2/\sigma^2)$ with variance $\sigma^2 \approx 0.1$.

3. The method of claim **2**, whereas basis $\{\mathbf{v}_d\}_{d=1}^{D}$ is obtained by some basis selection algorithm such that $D \approx 150$.

4. The method of claims **1** to **3**, whereas number of histological structures assumed to be present in [II] and [IV] is typically set to: $M \in \{4, 5, 6\}$.

5. The method of claims **1** to **4**, whereas nonnegativity and $\ell_0$-norm constrained matrix factorization algorithm is applied in [VI] to segment $M$ histological structures $\{\hat{\mathbf{s}}_m\}_{m=1}^{M}$.

**6.**

The method of claims **1** to **5** where digital staining (coloring) of segmented histological structures $\left\{\hat{\mathbf{s}}_m\right\}_{m=1}^{M}$ is performed according to linear mixture model [VI] with predefined color vectors $\left\{\mathbf{c}_m\right\}_{m=1}^{M}$ in RGB space.

**7.** The method of claims **1** to **6** where segmented histological structures $\left\{\hat{\mathbf{s}}_m\right\}_{m=1}^{M}$ and digitally stained image **Y are** stored and/or displayed on the output storing and/or display device.

**8.** The method of claim **1,** whereas the imaged specimen is a biological tissue sample.

**9.** The method of claim **8,** whereas the biological tissue comprises one or more abnormal histological structures.

**10.** The method of claim **9,** whereas said method is applied to discrimination and visualisation of at least two histological structures present in unstained biological tissue sample.

**11.** The method of claim **10,** whereas said method is applied to establish diagnosis of human disease such as: primary tumor of liver, kidney, lung, intestine and the like and also to detect metastatic invasion from a primary tumor.

**12.** The method of claim **1,** whereas said method is applied to: shortening slide preparation process, reducing intra-histologist variation in diagnosis, eliminating the possibility to add chemical effects to a specimen, eliminating the possibility to alter morphology of the specimen; simplifying histological and intra-surgical tissue analysis, enable multiple usage of the same specimen.

**Patentansprüche**

**1.** Ein Verfahren für die unüberwachte Segmentierung mikroskopischer Farbbilder von nicht verfärbten Proben und die digitale Färbung segmentierter histologischer Strukturen unter Verwendung der empirisch kernabbildungsbasierten nicht-linearen Abbildung eines aufgezeichneten mikroskopischen Bildes von unverfärbten Proben auf einen reproduzierbaren Kern des Hilbertraums, der Faktorisierung abgebildeter Bilder, eingeschränkt durch die Nicht-Negativität und die $l_0$ -Norm der binären {0,1} Quellen (histologische Strukturen) und die digitale Färbung von faktorisierten (segmentierten) histologischer Strukturen welches aus den folgenden Schritten besteht:

Aufzeichnung und Speicherung mikroskopischer Farbbilder von unverfärbten Proben *X,* wobei *X* eine Matrix von nicht-negativen Daten ist, die aus *N*=3 Zeilen besteht, die Graustufenbildern entsprechen, welche bei bestimmten Wellenlängen aufgezeichnet wurden, die den Farben Rot, Grün und Blau entsprechen und aus *T* Spalten, welche Beobachtungen verschiedener räumlicher Lagen (Pixel) entsprechen,
wobei die Matrix der Bilddaten durch das maximale Element von *X*, $\mathrm{X_{max}}$ dimensioniert wird:

$$X = X/\mathrm{x_{max}} \qquad \text{[I]}$$

wobei die Matrix der Bilddaten *X* durch ein lineares kombiniertes modell dargestellt ist:

$$X = AS \qquad \text{[II]}$$

wobei $A \in \mathbb{R}_{0+}^{3 x M}$ für eine nicht-negative Mischmatrix steht, bestehend aus M Spaltenvektoren $\left\{a_m\right\}_{m=1}^{M}$ die für Spektralprofile von M histologischen Strukturen stehen, die im Bild *X* vorhanden sind; *S* steht für eine binäre Quellmatrix *M* x *T* bestehend aus den Werten {0,1}, wobei das Element $\left\{S_{mt} \in \{0,1\}\right\}_{m,t=1}^{M,T}$ die

Anwesenheit (1) oder Abwesenheit (0) der histologischen Struktur *m* an der Pixelstelle *t* anzeigt.

- wobei eine empirische Kernabbildung zur nicht-linearen Abbildung von *X* in [II] auf einen reproduzierbaren

Kern des Hilbeitraums $\Psi$ (X) $\in \mathbb{R}_{0+}^{D \times T}$ verwendet wird:

$$\Psi(X) = \begin{bmatrix} K(\mathbf{x1}, \mathbf{v1}) & \cdots & K(\mathbf{xT}, \mathbf{v1}) \\ \cdots & \cdots & \cdots \\ K(\mathbf{x1}, \mathbf{vD}) & \cdots & K(\mathbf{xT}, \mathbf{vD}) \end{bmatrix} \qquad [\text{III}]$$

wo K $(x_1, v_d)$, $t = 1,..., T$ et $d = 1, D$ eine positive symmetrische Kernel-Funktion und $v_d$, $d = 1,..., D$ die Basisvektoren bezeichnet, die annähernd den gleichen Raum abdecken wie die Pixel-Vektoren : $x_t$, $t = 1,..., T$.
- wobei die abgebildete Matrix $\Psi(X)$ durch ein lineares kombiniertes Modell [IV] dargestellt ist:

$$\Psi(X) = BS \qquad [\text{IV}]$$

**dadurch gekennzeichnet, dass** S die gleiche binäre Quellmatrix wie in [II], während

$B \in \mathbb{R}_{0+}^{D \times T}$ die Matrizen in einem abgebildeten Raum mischen wobei die Spaltenvektoren

$\{b_m\}_{m=1}^{M}$ gegenseitig bedeutent weniger in Korrelation stehen als die Spaltenvektoren $\{a_m\}_{m=1}^{M}$

in [II], wodurch das Unterscheiden von spektral identischen histologischen Strukturen, die in Bild $X$ vorhanden sind, ermöglicht wird

wobei der durch Spärlichkeit und Nicht-Negativität eingeschränkte Matrixfaktorisierungsalgorithmus (*sNMF*) auf [IV] angewendet wird, wobei die Spärlichkeitseinschränkung anzeigenden Funktion von S basiert, wie die Quasi-Norm von S $\ell$ 0, um Schötzungen für die Anwesenheit/Abwesenheit der histologischen Strukturen

$\{S_m\}_{m=1}^{M}$ zu eimitteln:

$$\{a_m\}_{m=1}^{M} = sNMF(\Psi(X)) \qquad [\text{V}]$$

wo, wie in [II], $M$ die Anzahl der auf im Bild $X$ vorhandenen histologischen Strukturen bezeichnet;
- die segmentierte hiostologische Strukturen $\{\hat{S}m\}_{m=1}^{M}$ als schwarz-weisse Abbildungen anzeigen ;
- die segmentierte histologische Strukturen

mit $\{\hat{S}m\}_{m=1}^{M}$ vordefinierten Farben digital verfärben (färben) gemäß :

$$Y = C\hat{S} \qquad [\text{VI}],$$

wobei $\{C_m\}_{m=1}^{M}$ die vordefinierten Farbvektoren im RGB-Farbraum sind,
- die segmentierte histologische Strukturen als ein synthetisches Farbbild (RGB) Y anzeigt.

**2.** Ein Verfahren nach Anspruch **1, dadurch gekennzeichnet, dass** in der empirischen Kernabbildung [III] die positive symmetrische Kernfunktion ein invariant verschobener Kern ist: K $(x_t, v_d)$ = K $(x_t, v_d)$. Vorzugsweise, K $(x_t, v_d)$ ein Gaussscher Kern ist: K $(x_t, v_d)$ = exp (- $\|x_t - v_d\|^2/\sigma^2$) mit eine Varianz von $\sigma^2 \approx 0.1$.

**3.** Ein Verfahren nach Anspruch **2, dadurch gekennzeichnet, dass** die Basis $\{v_d\}_{d=1}^{D}$ durch einen Basis-Aus-

wahlalgorithmus erstellt wird, wobei D ≈ 150.

4. Ein Verfahren nach den Ansprüchen **1 bis 3, dadurch gekennzeichnet, dass** die angenommene Anzahl der vorhandenen histolologischen Strukturen in [II] und [IV] typischerweise auf $M \in \{4,5,6\}$ gesetzt wird.

5. Ein Verfahren nach den Ansprüchen **1 bis 4, dadurch gekennzeichnet, dass** Matrixfaktousierungsalgorithmus mit Einschränkungen durch die *l* 0-Norm und die Nicht-Negativität in [VI] auf M segmentierte histologische Strukturen $\{\hat{S}m\}_{m=1}^{M}$ angewendet wird.

6. Ein Verfahren nach den Ansprüchen **1 bis 5, dadurch gekennzeichnet, dass** die digitale Verfärbung (Färbung) segmentierter histologischer Strukturen $\{\hat{S}m\}_{m=1}^{M}$ gemäß einem linearen kombinierten Modell [VI] mit vorde-finierten Farbvektoren $\{c_m\}_{m=1}^{M}$ im RGB-Farbraum durchgeführt wird.

7. Ein Verfahren nach den Ansprüchen **1 bis 6, dadurch gekennzeichnet, dass** die segmentierten histologischen Strukturen $\{\hat{S}m\}_{m=1}^{M}$ und das digital gefärbte Bild **Y** auf dem Speicher- und/oder Anzeigegerät am Ausgang gespeichert und/oder angezeigt werden.

8. Ein Verfahren nach Anspruch 1, **dadurch gekennzeichnet, dass** die abgebildete Probe eine biologische Gewebeprobe ist.

9. Ein Verfahren nach Anspruch 8, **dadurch gekennzeichnet, dass** das biologische Gewebe eine oder mehrere abnormale histologische Strukturen enthält.

10. Ein Verfahren nach Anspruch 9, **dadurch gekennzeichnet, dass** das Verfahren zum Unterscheiden und zur Visualisierung von mindestens zwei, im unverfärbten biologischen Gewebe vorhandenen, histologischen Strukturen angewendet wird.

11. Ein Verfahren nach Anspruch 10, **dadurch gekennzeichnet, dass** das angegebene Verfahren für die Diagnose von Krankheiten bei Meschen angewendet wird, wie z.B. : primärer Tumor an Leber, Nieren, Lunge, Darm und ähnlichem und auch zur Erkennung eines metastatischen Eindringens seitens eines primären Tumors.

12. Ein Verfahren nach Anspruch **1, dadurch gekennzeichnet, dass** dass das angegebene Verfahren für folgendes angewendet wird : zur Abkürzung des Slide-Vorbereitungsverfahrens, Verringerung der intra-histologischen Vriationen bei der Diagnose, zur Verhinderung der Möglichkeit einer Probe chemische Effekte hinzuzufügen, zur Verhinderung der Möglichkeit, die Morfologie der Probe zu verändern, zur Vereinfachung der histologischen und intrchirurgischen Analyse von Geweben, und um die mehrfache Verwendung desselben Gewebes zu ermöglichen.

## Revendications

1. Une méthode de supervision non surveillée d'images couleur microscopiques de spécimens non colorés et coloration digitale de structures histologiques segmentées au moyen d'une modélisation non-linéaire à mappage de noyau empirique de l'image microscopique enregistrée de spécimens non colorés sur un noyau reproductible de l'espace de Hilbert, factorisation des images mappées limitée par la contrainte de non-négativité et la norme *l* 0 des sources (structures histologiques) binaires {0,1} et coloration digitale de structures histologiques factorisées (segmentées) comprenant les étapes suivantes:

enregistrement et stockage d'image couleur microscopique du spécimen non coloré *X*, où *X* est une matrice de données non-négatives formée de *N*=3 rangées correspondant aux images en échelle de gris enregistrées à certaines longueur d'onde correspondant aux couleurs rouge, vert et bleu et des colonnes *T* qui correspondent aux observations de différents endroits spatiaux (pixels), graduation de la matrice de données de l'image par

élément maximal $X$, $x_{max}$ :

$$X = X/x_{max} \qquad\qquad\qquad\qquad [\text{I}]$$

représentation de la matrice de données de l'image $X$ par modèle combiné linéaire :

$$X = AS \qquad\qquad\qquad\qquad [\text{II}]$$

dans lequel $A \in \mathbb{R}_{0+}^{3xM}$ correspond à une matrice non-négative combinée comprenant $M$ colonnes de vecteurs $\{a_m\}_{m=1}^{M}$ qui correspond aux profils spectraux de $M$ structures histologiques présentes dans l'image $X$ ; $S$ correspond à une matrice de source binaire $M$ x $T$ comprenant des valeurs {0,1} de façon à ce que l'élément $\{S_{mt} \in \{0,1\}\}_{m,t=1}^{M,T}$ indique la présence (1) ou l'absence (0) de la structure histologique $m$ à l'emplacement du pixel $t$.

- utilisant la carte de noyau empirique pour mappage non linéaire de $X$ dans [II] sur le noyau reproductible de l'espace de Hilbert $\Psi(X) \in \mathbb{R}_{0+}^{D\,x\,T}$ : $\Psi(X)$

$$\Psi(X) = \begin{bmatrix} K(\mathbf{x1}, \mathbf{v1}) & \cdots & K(\mathbf{x}T, \mathbf{v1}) \\ \cdots & \cdots & \cdots \\ K(\mathbf{x1}, \mathbf{v}D) & \cdots & K(\mathbf{x}T, \mathbf{v}D) \end{bmatrix} \qquad [\text{III}]$$

dans lequel K $(x_1, v_d)$, $t = 1,..., T$ et $d = 1, D$ correspond à la fonction de noyau symétrique positive et $v_d$, $d = 1,..., D$ correspondent aux vecteurs de base qui couvrent approximativement le même espace que les vecteurs de pixels : $x_t$, $t = 1,..., T$.
- représentant la matrice mappée $\Psi(X)$ par le modèle combiné linéaire [IV] :

$$\Psi(X) = BS \qquad\qquad\qquad\qquad [\text{IV}]$$

**caractérisée en ce que** S est la même matrice de source binaire que dans [II], ou $B \in \mathbb{R}_{0+}^{D\,x\,T}$ mélange les matrices dans l'espace mappé afin que les vecteurs de colonnes $\{b_m\}_{m=1}^{M}$ soient mutuellement considérablement moins corrélés que les vecteurs de colonnes $\{a_m\}_{m=1}^{M}$ dans [II], permettant de discriminer les structures histologiques spectralement similaires présentes dans l'image $X$,

appliquant l'algorithme de factorisation de matrice contraint par la rareté et la non-négativité (*sNMF*) à [IV] alors que la contrainte de rareté repose sur l'indicateur de fonction de S **en ce que** la quasi-norme de S $\ell 0$, afin d'obtenir des estimations de présence/absence de structures histologiques $\{S_m\}_{m=1}^{M}$ :

$$\{a_m\}_{m=1}^{M} = sNMF(\Psi(X)) \qquad\qquad [\text{V}]$$

dans lequel comme dans [II], *M* désigne le nombre de structures histologiques présentes dans l'image *X* ;

- en affichant les structures histologiques segmentées $\left\{ \hat{S}m \right\}_{m=1}^{M}$ en tant que cartes en noir et blanc ;

- en teintant (colorant) digitalement les structures histologiques segmentées $\left\{ \hat{S}m \right\}_{m=1}^{M}$ avec des couleurs prédéfinies selon :

$$Y = C\hat{S} \qquad\qquad [VI],$$

dans lequel $\left\{ C_m \right\}_{m=1}^{M}$ correspond aux couleurs prédéfinies des vecteurs dans le schéma de couleurs RVB,

- en affichant les structures histologiques segmentées comme image Y à couleurs synthétiques RVB.

2. La méthode selon la revendication **1, caractérisée en ce que** dans la carte de noyau empirique [III] la fonction de noyau symétrique positive est un noyau à déplacement invariant : K $(x_t, v_d)$ = K $(x_t, v_d)$. Préférablement, K $(x_t, v_d)$ est un noyau Gaussien : K $(x_t, v_d)$ = exp $(-\|x_t - v_d\|^2/(\sigma^2)$ avec une variance de $\sigma^2 \approx 0.1$.

3. La méthode selon la revendication 2, **caractérisée en ce que** la base $\left\{ v_d \right\}_{d=1}^{D}$ est obtenue par une sélection basique d'algorithme telle que D $\approx$ 150.

4. La méthode selon les revendications **1 à 3 caractérisée en ce que** le nombre de structures histologiques supposé présentes en [II] et [IV] est typiquement appliqué à $M \in \{4,5,6\}$.

5. La méthode selon les revendications **1 à 4 caractérisée en ce que** l'algorithme de factorisation de matrice avec les contraintes de norme *l* 0 et de non-négativité est appliqué dans [VI] à M structures histologiques $\left\{ \hat{S}m \right\}_{m=1}^{M}$.

6. La méthode selon les revendications **1 à 5 caractérisée en ce que** la teinture (coloration) digitale des structures histologiques segmentées $\left\{ \hat{S}m \right\}_{m=1}^{M}$ est réalisée selon un modèle mixte linéaire [VI] avec des vecteurs de couleurs prédéfinis $\left\{ c_m \right\}_{m=1}^{M}$ dans l'espace RVB.

7. La méthode selon les revendications **1 à 6 caractérisée en ce que** les structures histologiques segmentées $\left\{ \hat{S}m \right\}_{m=1}^{M}$ et l'image **Y** colorée digitalement sont stockées et/ou affichées sur le dispositif de sortie de stockage et/ou affichage.

8. La méthode selon la revendication **1 caractérisée en ce que** l'image spécimen est un échantillon de tissu biologique.

9. La méthode selon la revendication **8 caractérisée en ce que** le tissu biologique contienne une structure histologique anormale voire plus.

10. La méthode selon la revendication 9 **caractérisée en ce que** la méthode est appliquée à la discrimination et la visualisation d'au moins deux structures histologiques présentes dans un échantillon de tissu biologique non permanent.

11. La méthode selon la revendication 10 **caractérisée en ce que** ladite méthode est appliquée au diagnostique établi de maladies humaines comme : tumeur primaire du foie, reins, poumons, intestins ou similaires et également pour détecter l'invasion métastatique à partir d'une tumeur primaire.

12. La méthode selon la revendication 1 **caractérisée en ce que** ladite méthode est appliquée à : réduction du processus d'élaboration des pages, réduction des variations intra-histologiques dans les diagnostiques, élimination de la possibilité d'ajouter des effets chimiques à un spécimen, élimination de la possibilité de modifier la morphologie du spécimen, simplification de l'analyse histologique et intra-chirurgicale des tissus, possibilité d'utilisation multiple du même spécimen.

FIG. 1

FIG. 2A

FIG. 2B

FIG. 2C

**FIG. 2D**

**FIG. 2E**

FIG. 3A

FIG. 3B

**FIG. 3C**

**FIG. 3D**

**FIG. 3E**

**FIG. 4A**

**FIG. 4B**

**FIG. 4C**

**FIG. 4D**

**FIG. 4E**

**FIG. 5A**

**FIG. 5B**

**FIG. 5C**

**FIG. 5D**

FIG. 5E

FIG. 5F

**FIG. 5G**

**FIG. 6A**

**FIG. 6B**

**FIG. 6C**

31

**FIG. 6D**

**FIG. 6E**

**FIG. 6F**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8532376 B
- US 20130071002 A
- US 20120269417 A
- US 20110026803 A
- US 20070016081 A
- WO 2011078980 A
- CN 101667299
- US 2010111382 A
- US 2013317369 A

- US 20110228072 A
- US 20110134233 A
- US 2011005817 A
- US 20110058177 A
- US 20080032325 A
- US 20070109874 A
- JP H0225251 B
- CA 1036385
- US 20120147002 A

**Non-patent literature cited in the description**

- **D. MARR ; E. HILDREDTH.** Theory of Edge Detection. *Proc. Royal Soc London Ser B Biol Sci,* 1980, vol. 207, 187-217
- **K. DIAMANTARAS ; T. PAPADIMITROU ; G. VRANOU.** Blind separation of multiple binary sources from one nonlinear mixture. *Proc. IEEE Int. Conf. Acoust. Speech and Sig. Proc. (ICASSP-2011),* 2011, 2108-2111
- **K. DIAMANTARAS.** Blind separation of multiple binary sources using a single linear mixture. *Proc. IEEE Int. Conf. Acoustic, Speech and Signal Processing (ICASSP-2000),* June 2000, 2889-2892
- A Clustering Approach for Blind Separation of Multiple Finite Alphabet Sequences from a Single Linear Mixture. **K. I. DIAMANTARAS.** Signal Processing. Elsevier, April 2006, vol. 86, 877-891
- **K. I. DIAMANTARAS ; T. PAPADIMITRIOU.** Separating two binary sources from a single nonlinear mixture. *Proc. Int. Conf. Acoustics, Speech and Signal Processing (ICASSP 2010),* 14 March 2010, 1946-1949
- **K. I. DIAMANTARAS ; T. PAPADIMITRIOU.** Blind separation of three binary sources from one nonlinear mixture. *Proc. 2010 Int. Workshop on Machine Learning for Signal Processing (MLSP-2010),* 29 August 2010, 301-306
- **M. CASTELLA.** Inversion of Polynomial Systems and Separation of Nonlinear Mixtures of Finite-Alphabet Sources. *IEEE Trans. on Sig. Proc.,* August 2008, vol. 56 (8), 3905-3917
- **Y. LI ; A. CICHOCKI ; L. ZHANG.** Blind separation and extraction of binary sources. *IEICE Trans. Fundamentals,* March 2003, vol. E86-A (3), 580-589

- **K. I. DIAMANTARAS ; T. PAPADIMITRIOU.** Blind deconvolution of multi-input single-output systems with binary sources. *IEEE Trans. Signal Process.,* October 2006, vol. 54 (10), 3720-3731
- **K. I. DIAMANTARAS ; E. CHASSIOTI.** Blind separation of n binary sources from one observation: A deterministic approach. *Proc. Int. Conf. Independent Component Analysis and Signal Separation (ICA),* June 2000
- **Q. DU ; I. KOPRIVA ; H.SZU.** Independent Component Analysis for Classifying Multispectral Images with Dimensionality Limitation. *International Journal of Information Acquisition,* September 2004, vol. 1 (3), 201-216
- Nonlinear Band Expansion and Nonnegative Matrix Underapproximation for Unsupervised Segmentation of a Liver from a Multi-phase CT image. **I. KOPRIVA ; X. CHEN ; Y. JAO.** SPIE Medical Imaging-Image Processing, Orlando, FL, USA. Proc. SPIE, 12 February 2011, vol. 7962, 79623A-1, 79623A-8
- Nonlinear Band Expansion and 3D Nonnegative Tensor Factorization for Blind Decomposition of Magnetic Resonance Image of the Brain. **I. KOPRIVA ; A. CICHOCKI.** Proc. of 9th Int. Conf. on Latent Variable Analysis and Signal Separation. Lecture Notes Computer Science, 27 September 2010, vol. 6365, 490-497
- **OUYANG, Y. C. ; CHEN, H. M. ; CHAI, J. W. ; CHEN, C. C. C. ; POON, S. K. ; YANG, C. W. ; LEE, S. K. ; CHANG, C. I.** Band Expansion-Based Over-Complete Independent Component Analysis for Multispectral Processing of Magnetic Resonance Image. *IEEE Trans. Biomed. Eng.,* 2008, vol. 55, 1666-1677

- **R. PEHARZ ; F. PERNKOPF.** Sparse nonnegative matrix factorization with $\ell 0$ constraints. *Neurocomputing,* 2012, vol. 80, 38-46